# EUROPEAN PATENT APPLICATION

(11) **EP 2 160 991 A2**
(43) Date of publication of application: **10.03.2010**
(21) Application number: 09460040.0
(22) Date of filing: 05.09.2009
(51) Int. Cl.: A61B 18/02

(54) **Liquid nitrogen cryosurgical apparatus with a set of Suwalski cardiac surgery cryoprobes**

(30) Priority: 07.09.2008 PL 11763408
(71) Applicant: Kriomedpol Spólka z o. o., 05-082 Stare Babice (PL)
(72) Inventor: Policewicz, Bogdan, 02-571 Warszawa (PL); Narkiewicz, Tadeusz, 01-355 Warszawa (PL); Karaskiewicz, Andrzej, 05-080 Koczaragi Stare (PL); Suwalski, Kazimierz, 04-216 Warszawa (PL); Suwalski, Piotr, 02-593 Warszawa (PL)
(74) Representative: Czub, Krzysztof

(57) **Abstract**

The liquid nitrogen cryosurgical apparatus with a set of Suwalski cardiac surgery cryoprobes comprises of the liquid nitrogen container (1) installed on the mobile frame (11) comprising of a base and a profiled extension arm. The container (1) cooperates with the supply head (5) which is detachably connected to the supply line (2) by means of connector (7) fastening the line (2) to the head (5). The cooling medium used is the liquid nitrogen which is transported from the container (1) through the supply line (2) and is supplied to the selected Suwalski cardiac surgery cryoprobe (4) tip seated in the socket inside the handle (3) of the supply line (2). The supply line (2) comprises of four coaxially arranged flexible hoses, and the first internal hose (14) supplies the liquid nitrogen. The electrical system of the supply head (5) is connected by means of the power supply connector (8), located on the wiring harness end, with the control panel (6) installed on the mobile frame (11) extension arm. The line (2) handle (3) holder (12) is also attached to the extension arm of the said frame (11). On the base of the mobile frame (11) the power switch (9) with a network cable socket, fuses (10) and the supply head (5) holder (13) are located. On the end of the supply line (2) a teflon locking sleeve (15) is located. One end of the sleeve (15) has a cryoprobe teflon socket (16, 17) attached, whereas on the other end a teflon mounting socket (19) of the first internal hose (14) is located.

## Description

Subject to the invention /utility model/ is the liquid nitrogen cryosurgical apparatus with a set of Suwalski cardiac surgery cryoprobes, designed mainly for local freezing of a tissue during surgery, in particular during the open-heart surgery. In order to assure fast and effective freezing of a diseased tissue, the cooling medium used is liquid nitrogen with a temperature of-196°C; the tissue freezes upon contact with a cold operating tip of the cryoprobe.

The liquid nitrogen cryosurgical apparatus with one mobile frame-mounted liquid nitrogen container is known. The container is designed to cooperate with a head which is detachably connected to a line supplying the liquid nitrogen to the cryoprobe located in a handle. Electrical system of the head is connected to a frame-mounted control panel by means of a connector located on the wiring harness end. The head is equipped with a pressure regulator, two safety valves, a solenoid valve which cuts off the liquid nitrogen flow, and a heater with a safety device which maintains a proper pressure in the container. The heater is attached to the bottom part of the head assembly, so that after attaching the assembly to the container, it is favourably placed 1 cm above the bottom of the container. The supply line comprises of four coaxially arranged flexible hoses of different diameters; the first internal hose supplies the liquid nitrogen. On the end of the line a handle with the cryoprobe is located. The apparatus is controlled by means of a control and power supply panel installed on the mobile frame. On the control panel the head assembly power supply connector, a temperature regulator switch, and a continuous cooling system switch are located; above the switches the diodes are located. On the face of the panel the liquid nitrogen level indicator, the temperature regulator, and a power switch with a 'power on' diode are also located.
A shortcoming of this apparatus is its inability to be used in case of the open-heart surgery. The apparatus has low cooling efficiency and insufficient precision of the liquid nitrogen level indication system. The known solution does not guarantee that the cryoprobe is completely free of voltage, even the residual voltage.

The invention /utility model/ of the apparatus comprises of a liquid nitrogen container installed on the mobile frame comprising of a base and a profiled extension arm. On the extension arm a control panel and a line handle holder are located; the power supply switch, a network cable socket, fuses, and the head holder are located on the base. On the container there is installed a supply head which is detachably connected through a connector to the line supplying the liquid nitrogen to one of Suwalski cardiac surgery cryoprobes located inside the supply line handle. The supply line comprises of four coaxially arranged flexible hoses; the first internal hose, having the diameter of A, supplies the liquid nitrogen. On the end of the supply line a teflon locking sleeve is installed. One end of the sleeve has a teflon probe socket attached, whereas on the other end a teflon mounting socket for the first internal hose is located. The probe socket consists of two parts: the replaceable external part comprising the B-diameter channel, and the internal part comprising the A-diameter channel; between these parts a socket fastener is located. The mounting socket of the first hose comprises the fastener having the diameter of A. The sockets are seated inside the sleeve coaxially, within such a distance that the cryoprobe and the hose fastener do not come into contact; between these elements a liquid nitrogen teflon supply channel, having the diameter of A, is located. The teflon sleeve is installed in the supply line handle, to which the cryoprobe is attached.
The set of Suwalski cardiac surgery cryoprobes comprises of five cryoprobes, each consisting of a fastening section, an insulating section that also ensures proper manoeuvrability during a surgery, and an operating tip. Each of fastening sections is seated inside the locking sleeve socket and has identical cross-section and shape. The fastening section includes two sleeves, one of which has the diameter of B, and the second one, located at the end of a cryoprobe, has the diameter of A, that is the diameter equal to the diameter of the fastener of the supply line first internal hose. The fastening section is seated in the probe socket and is ended with two rings, the difference between external radiuses of which is slight. The first cryoprobe operating tip is shaped as a scrap of an arc and is slightly deflected. The second cryoprobe tip is shorter than the first cryoprobe tip and is shaped as a scrap of an arc. The third cryoprobe tip has larger diameter than the other tips and is bent in relation to the insulating section. The fourth cryoprobe tip is shaped as a scrap of an arc and is longer than the first and second cryoprobe tips. The fifth cryoprobe tip is T-shaped.
The apparatus control panel is cuboid-shaped. On the panel face a cryoprobe digital temperature meter and two diodes are located; upper diode indicates lack of nitrogen in the container, and the other one (lower) indicates readiness of the apparatus for operation. In the upper right part of the panel a digital nitrogen level meter is located; below a thermoregulatory system switch with a diode placed to its right side, and a continuous cooling system switch with a diode placed to its left side are situated. On the panel sidewall a head power supply socket is located, and a panel power supply socket is installed on the base. In the base of the mobile frame a tensometer, a balance amplifier, and two transformers are installed, one of which supplies the balance circuit, and the second one supplies the control panel.

The utility model of the apparatus due to having a set of cardiac surgery cryoprobes with specifically designed tips allows to freeze a tissue during the open-heart surgery. The fastening method of the cryoprobe inside the supply line holder by means of the teflon sleeve prevents its contact with any metal elements, and thus ensures that the cryoprobe tip is completely free of voltage (even the residual voltage). The tensometric balance and the digital indicator ensure required high working precision of the liquid nitrogen level measuring system is reached which greatly improves the work comfort of a surgeon performing cardiac surgery by providing accurate information on the level of the liquid nitrogen in the container.

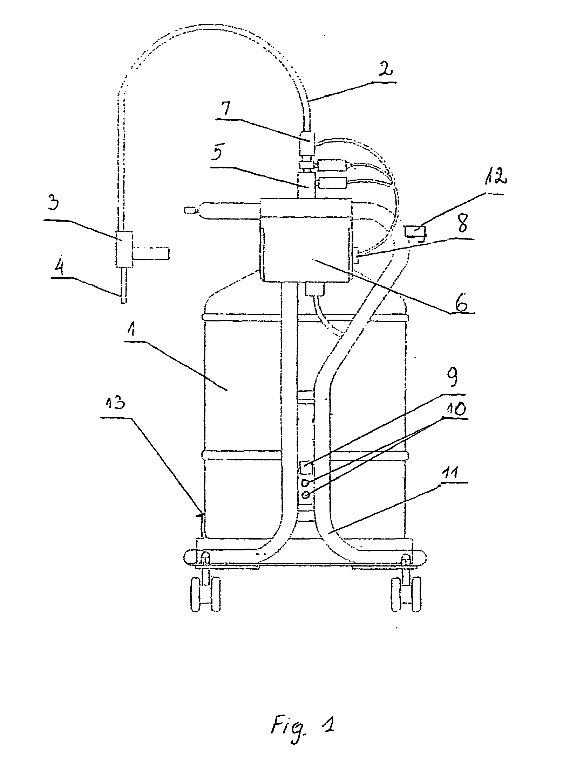

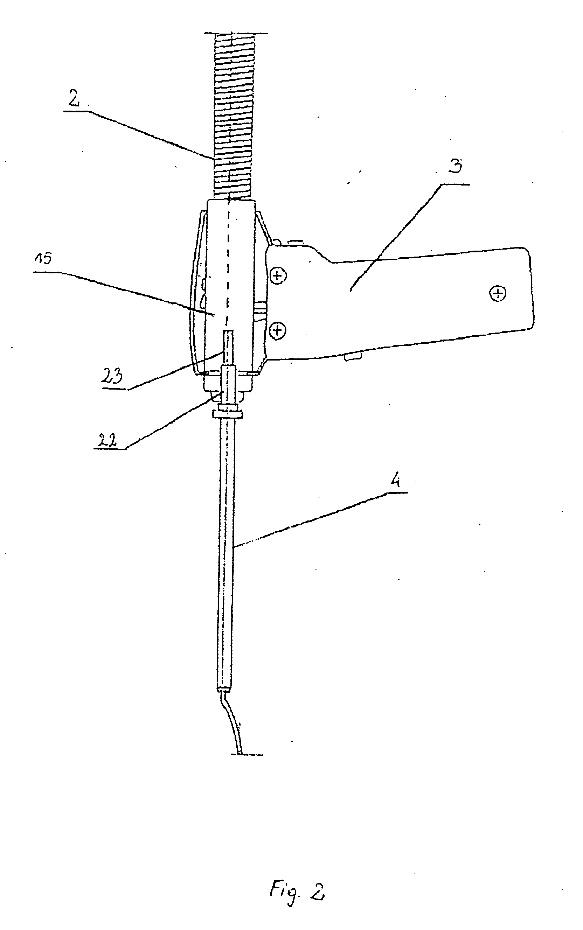

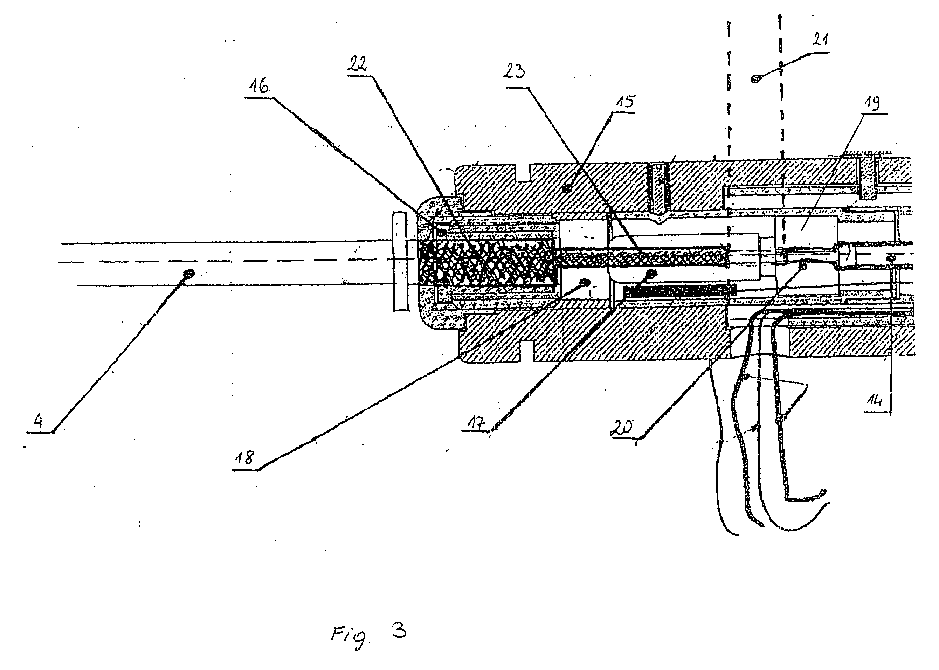

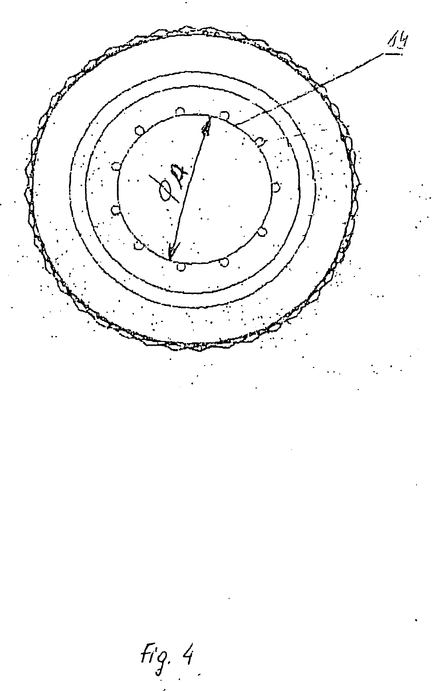

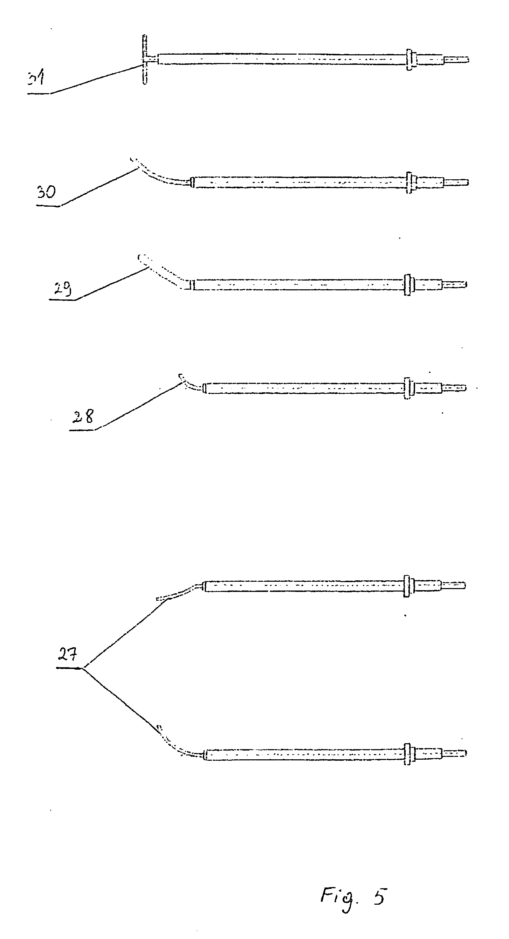

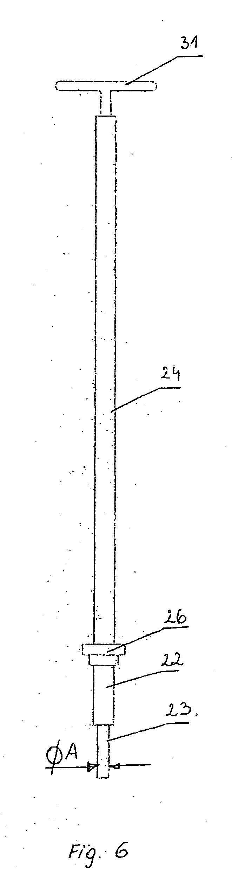

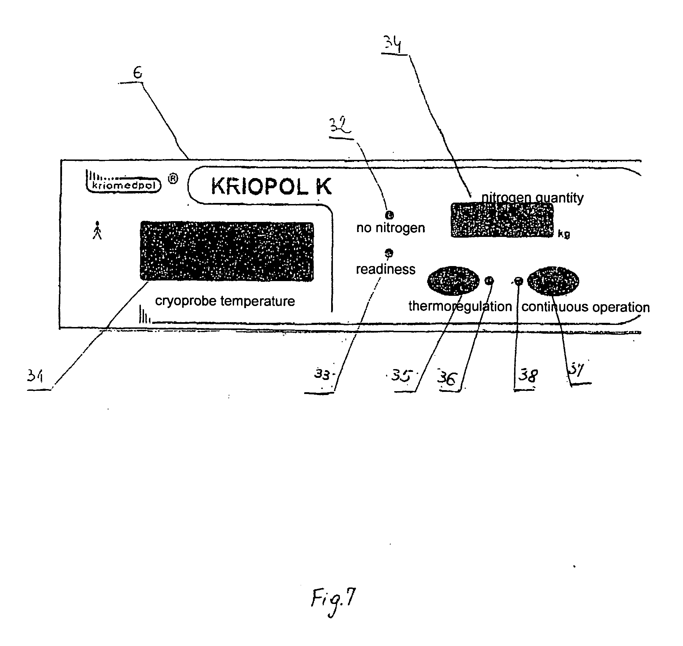

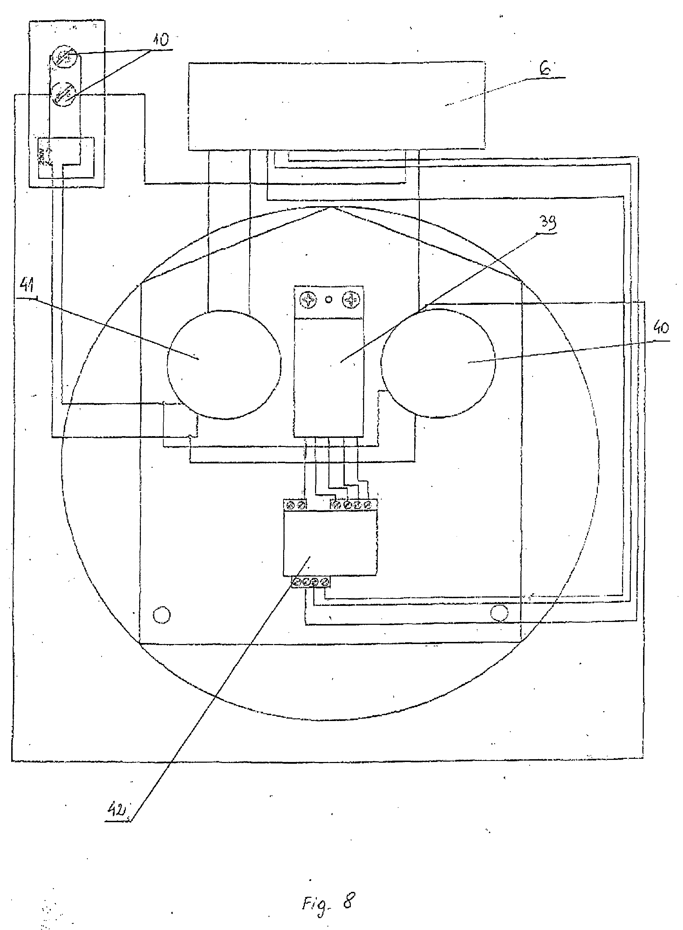

The utility model of the apparatus is shown in the example drawing, where fig. 1 shows a general view of the apparatus, fig. 2 - the cryoprobe supply line handle, fig. 3 - the teflon locking sleeve cross-section, fig. 4 - the supply line cross-section, fig. 5 - the set of Suwalski cardiac surgery cryoprobes, fig. 6 - the cryoprobe with T-shaped tip, fig. 7 - the control panel face, fig. 8 - the balance assembly.

The apparatus has the liquid nitrogen container (1) installed on the mobile frame (11) comprising of a base and a profiled extension arm. The container (1) cooperates with the supply head (5) which is detachably connected to the supply line (2) by means of connector (7) fastening the line (2) to the head (5). The cooling medium used is the liquid nitrogen which is transported from the container (1) through the supply line (2) and is supplied to the selected Suwalski cardiac surgery cryoprobe (4) tip seated in the socket inside the handle (3) of the supply line (2). The supply line (2) comprises of four coaxially arranged flexible hoses, and the first internal hose (14), having the diameter of A, transports the liquid nitrogen. The electrical system of the supply head (5) is connected by means of the power supply connector (8), located on the wiring harness end, with the control panel (6) installed on the mobile frame (11) extension arm. The line (2) handle (3) holder (12) is also attached to the extension arm of the said frame (11). On the base of the mobile frame (11) the power switch (9) with a network cable socket, fuses (10) and the supply head (5) holder (13) are located. On the end of the supply line (2) a teflon locking sleeve (15) is located. One end of the sleeve (15) has a cryoprobe teflon socket (16, 17) attached, whereas on the other end a teflon mounting socket (19) of the first internal hose (14) is located. The probe socket (16, 17) consists of two parts: the replaceable external part (16) comprising the B-diameter channel, and the internal part (17) comprising the A-diameter channel; between these parts a socket fastener (18) is located. The first hose (14) mounting socket (19) comprises a fastener (20) having the diameter of A. The sockets (16, 17) are seated inside the sleeve (15) coaxially, and within such a distance that the cryoprobe (4) and the hose (14) fastener (20) do not come into contact; between these elements a liquid nitrogen teflon supply channel (21) having the diameter of A is located. The teflon sleeve (15) is installed in the supply line (2) handle (3), to which the cryoprobe (4), selected by a surgeon performing cardiac surgery, is attached.
The set of Suwalski cardiac surgery cryoprobes comprises of five cryoprobes, each consisting of a fastening section (22, 23) and the insulating section (24) which ensures also a proper manoeuvrability during a surgery, and the operating tip (25). Each of the fastening sections (22, 23) is seated inside the locking sleeve (15) socket and has identical cross-section and shape. The fastening section (22, 23) includes two sleeves: one sleeve (22) has the diameter of B, and the other sleeve (23), located at the cryoprobe (4) end, has the diameter of A, that is the diameter equal to the diameter of the fastener (20) of the supply line (2) first internal hose (14). The fastening section (22, 23) of the cryoprobe (4) is seated in the socket (16, 17), and is ended with two rings (26), the difference between external radiuses of which is slight. Each of Suwalski cardiac surgery cryoprobes has a different operating tip. The first cryoprobe (27) tip is shaped as a scrap of an arc and is slightly deflected. The second cryoprobe (28) tip is shorter than the first cryoprobe (27) tip and is shaped as a scrap of an arc. The third cryoprobe (29) tip has larger diameter than the other tips and is bent in relation to the insulating section (24). The fourth cryoprobe (30) tip is shaped as a scrap of an arc and is longer than the first and second cryoprobe (27, 28) tips. The fifth cryoprobe (31) tip is T-shaped.
The apparatus control panel (6) has the shape of a cuboid. On the panel face a cryoprobe (31) digital temperature meter and two diodes are located. Upper diode (32) indicates lack of nitrogen in the container, and the lower diode (33) indicates readiness of the apparatus for operation. In the upper right part of the panel (6) a digital nitrogen level meter (34) is located, and below a thermoregulatory system switch (35) with the diode (36) placed to the right side of the switch (35), and a continuous cooling system switch (37) with a diode (38) placed to its left side are situated. In the base of the mobile frame (11) a tensometer (39), a balance amplifier (42), and two transformers are installed. One transformer (40) supplies the balance circuit, and the second transformer (41) supplies the apparatus control panel (6).

## Claims

1. The liquid nitrogen cryosurgical apparatus with a set of Suwalski cardiac surgery cryoprobes comprising the liquid nitrogen container, mounted on the mobile frame, which cooperates with the head detachably connected to the supply line comprises of four coaxially arranged flexible hoses; the first internal hose supplies the liquid nitrogen to the cryoprobe tip located inside the handle; the apparatus is controlled by means of a control and power supply panel equipped with indicators, switches, and diodes, **characterised in that** the supply head (5) is connected to the supply line (2) by means of connector (7); on the end of the supply line (2), inside the handle (3), a locking sleeve (15) is located; one end of the sleeve has a cryoprobe socket (16, 17) attached, whereas on the other end a mounting socket (19) of the first internal hose (14) is located; in the cryoprobe socket (16, 17) one of the five Suwalski cardiac surgery cryoprobes is installed; the apparatus is controlled by the control panel (6) installed on the mobile frame (11) extension arm, to which also the holder (12) of the supply line (2) handle (3) is attached; on the base the power switch (9) with a network cable socket, fuses (10) and the supply head (5) holder (13) are located; moreover, in the frame (11) base, below the container (1), a balance assembly is installed.

2. The apparatus, according to the claim 1, **characterised in that** the supply line (2) is ended with a teflon locking sleeve (15), to one side of which a cryoprobe teflon socket (16, 17) is attached, and on the other side a teflon mounting socket (19) of the first internal hose (14) is located; the probe socket (16, 17) consists of two parts: the replaceable external part (16) comprising the B-diameter channel, and the internal part (17) comprising the A-diameter channel; between these parts a socket fastener (18) is located, and the first hose (14) mounting socket (19) comprises a fastener (20) having the diameter of A; the sockets (16, 17) are seated inside the sleeve (15) coaxially, and within such a distance that the cryoprobe (4) and the hose (14) fastener (20) do not come into contact; between these elements a liquid nitrogen teflon supply channel (21) having the diameter of A is located.

3. The apparatus, according to the claim 1, **characterised in that** the set of Suwalski cardiac surgery cryoprobes comprises of five cryoprobes, each consisting of a fastening section (22, 23) and the insulating section (24) which ensures a proper manoeuvrability during a surgery, and the operating tip (25); each of the fastening sections (22, 23) is seated inside the locking sleeve (15) socket and has identical cross-section and shape; the fastening section (22, 23) includes two sleeves: one sleeve (22) has the diameter of B, and the other sleeve (23), located at the cryoprobe (4) end, has the diameter of A, that is the diameter equal to the diameter of the fastener (20) of the supply line (2) first internal hose (14); the fastening section (22, 23) of the cryoprobe (4) is seated in the socket (16, 17), and is ended with two rings (26), the difference between external radiuses of which is slight.

4. The apparatus, according to the claim 1, **characterised in that** each of Suwalski cardiac surgery cryoprobes has a different operating tip: the first cryoprobe (27) tip is shaped as a scrap of an arc and is slightly deflected, the second cryoprobe (28) tip is shorter than the first cryoprobe (27) tip and is shaped as a scrap of an arc, the third cryoprobe (29) tip has larger diameter than the other tips and is bent in relation to the insulating section (24), the fourth cryoprobe (30) tip is shaped as a scrap of an arc and is longer than the first and second cryoprobe (27, 28) tips, and the fifth cryoprobe (31) tip is T-shaped.

5. The apparatus, according to the claim 1, **characterised in that** the apparatus control panel (6) has the shape of a cuboid; on the panel face a cryoprobe (31) digital temperature meter and two diodes are located; upper diode (32) indicates lack of nitrogen in the container, and the lower diode (33) indicates readiness of the apparatus for operation; in the upper right part of the panel (6) a digital nitrogen level meter (34) is located, and below a thermoregulatory system switch (35) with the diode (36) placed to the right side of the switch (35), and a continuous cooling system switch (37) with a diode (38) placed to its left side are situated.

6. The apparatus, according to the claim 1, **characterised in that** in the base of the mobile frame (11) a tensometer (39), a balance amplifier (42), and two transformers are installed; one transformer (40) supplies the balance circuit, and the second transformer (41) supplies the apparatus control panel (6).
